(19) Europäisches Patentamt
European Patent Office
Office européen des brevets

(11) Publication number: **0 131 612**
**B1**

# (12) EUROPEAN PATENT SPECIFICATION

(45) Date of publication of patent specification: **12.11.86**

(51) Int. Cl.$^4$: **A 61 M 16/00**

(21) Application number: **84900527.7**

(22) Date of filing: **13.01.84**

(86) International application number:
**PCT/HU84/00002**

(87) International publication number:
**WO 84/02657 19.07.84 Gazette 84/17**

## (54) IMPROVED TRACHEA CANNULA.

(30) Priority: **14.01.83 HU 12483**

(43) Date of publication of application:
**23.01.85 Bulletin 85/04**

(45) Publication of the grant of the patent:
**12.11.86 Bulletin 86/46**

(84) Designated Contracting States:
**AT BE CH DE FR GB LI LU NL SE**

(56) References cited:
**DE-A-2 505 123**
**US-A-3 169 529**

(73) Proprietor: **LICHTENBERGER, György**
**Pozsonyi ut 15-17. V.I.**
**H-1137 Budapest (HU)**

(72) Inventor: **LICHTENBERGER, György**
**Pozsonyi ut 15-17. V.I.**
**H-1137 Budapest (HU)**

(74) Representative: **Patentanwälte Viering & Jentschura**
**Steinsdorfstrasse 6**
**D-8000 München 22 (DE)**

**EP 0 131 612 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to trachea cannula comprising a curved outer tube insertable into the trachea of a patient through a neck hole that is opened surgically by laryngotracheotomy, an inner tube slidably an removably fitted within the outer tube, and a box-like housing provided with a rear wall facing the patient's chest when the trachea cannula is in use , a front wall and bottom, top and side walls, and formed with at leat one breathing hole and connected to the open outer end of the inner tube.

There are a large number of patients suffering from various kind of throat trouble, such as lryngitis, cancer of the larynx etc. whose life has been saved by surgical treatment, especially by a total removal of the larynx. Such people can survive the operation by 5 to 10 or even 20 years. However, as a result of said operation they are obliged to use trachea cannula, a device as indicated briefly above, and through this they are put to great inconvenience. It is vital that they should use the trachea cannula, otherwise the neck hole made by laryngotracheotomy for breathing will narrow, and this may lead to suffocation.

The known prior art trachea cannulae used all over the world save the patients from suffocation. It is a disadvantage, however, that the outer end of the inner tube of such devices opens directly into the surroundings of the patient, and when they cough, secretion of the trachea is discharged into the ambient air, endangering the health of other people, and causing disgust. Because of reflex-like, spontaneous, sudden coughing the patients are not able to cover the free opening of the cannula with their handkerchiefs in time. Especially soon after the operation, or in case of patients who had been suffering from chronic tracheal catarrh, the discharge of secretion may be extremely intensive, and this puts them and people in their surrounding to increased inconvenience. Experience has shown that the quality of life of patients who have been cured from their basic throat disease by surgial treatment as described above, is poor. They often develop depression, and may exclude themselves from social contacts.

A known trachea cannula of the kind described in the preamble part of claim 1 (DE—A—2 505 123) is intended for use by a patient whose larynx is at least in part preserved. The box-like housing is a valve housing of a speaking valve flap closing upon expiring to direct the air stream through the larynx to enable the patient to speak. For a free breathing, the valve housing is moved into an inactive position in which the outer end of the inner tube is freely exposed.

The object of the present invention is to provide a trachea cannula which, in addition to enabling the patient to breathe freely, prevents infectious or merely disgusting secretion from being discharged into the ambient air.

The above object is achieved by a trachea cannula of the type described in the preamble part of claim 1, in which, according to the present invention, the box-like housing is constructed as a receptable adapted for collection of secretion and having closed front wall, the breathing hole, which is formed as a free throughhole, being located in the rear wall or at least one side wall of the receptacle which either is of the disposable kind and simply removably attached to the inner tube or is integrally formed with the inner tube as a single disposable component part.

In the embodiment in which the receptacle is simple removably attached to the inner tube, a coupling hole for the open outer end of the inner tube can be cut out from the rear wall of the receptacle so as to match the open outer end of the inner tube at least partially both in shape and dimension, the coupling hole being located in near proximity of the breathing hole.

With other embodiments, there are two breathing holes in the rear wall of the receptacle, arranged at both sides close to the coupling hole in a manner that the breaching holes and the coupling hole form a single shaped opening within the rear wall of the receptacle.

It has turned out to be advantageous if the trachea cannula according to the invention has also fixing means for securely but releasably attaching the receptacle to the open outer end part of the inner tube. In certain embodiments, each of the outer and inner tubes may have flanges at their open outer ends. The fixing means is preferably constructed as a snap fastener comprising an annular rim section of the flange of the inner tube to be inserted under slight pressure against the action of a ring-shaped wire spring into a matching bore section within the flange of the outer tube to securely clamp portions of the rear wall of the receptacle around the coupling hole between the flanges of the inner and outer tubes.

It is strongly recommended to use disposable thin-wall receptacles made of plastic material. In order to prevent deformation of the receptacle's front wall during breathing-in, at least a pair of distance pins may project from the flange of the inner tube towards the front wall of the receptacle.

As already mentioned, soon after the operation, or in case of patients who had been suffering from chronic tracheal catarrh, the discharge of secretion may be extremely intensive. In such mainly transitory cases, preferred embodiments of the trachea cannula according to the invention have proved to be very useful wherein there is a pipe of reduced diameter arranged within the inner tube, the pipe having an inlet portion in the proximity of the lower inner end of the inner tube, and a pipe end extending out of the receptacle. The pipe end is suitable for being connected to a sucking device, preferably a secretion pump, for positively removing excess secretion from the patient's trachea. In a further preferred embodiment the trachea cannula may also be equipped with a pipe that extends into the receptacle and

has an inlet portion arranged within a suitable distance above the bottom wall of the same. The pipe has a pipe end outside the receptacle for attaching a sucking device, preferably a secretion pump, thereto for a temporary and positive discharge of the contents of the receptacle.

With all embodiments of the trachea cannula in accordance with the present invention, shape and dimension of the receptacle may show a large variety. Designs of prismatic shape, disc-like embodiments or even oval ones may equally be chosen. Different sizes or receptacles may be of particular advantage too, in accordance with prevailing criteria. However, it is of fundamental importance that the receptacle applied should be able to prevent uncontrolled discharge of secretion into the patient's surroundings.

In the following, the present invention will be more particularly described, by way of example only, through preferred embodiments with reference to the drawings wherein

Fig. 1 shows a diagrammatic perspective view of a first preferred embodiment of a trachea cannula according to the invention,

Fig. 2 shows the perspective view of the disposable receptacle of the embodiment indicated in Fig. 1,

Fig. 3 indicates a cross-sectional longitudinal view of the embodiment according to Fig. 1 wherein details of a preferred fixing means are more clearly shown in a somewhat enlarged scale, and

Fig. 4 shows the same cross-sectional longitudinal view of a further embodiment of the trachea cannula according to the invention where the inner tube and the receptacle are formed as a single disposable component part made entirely of plastics material.

A first preferred embodiment of trachea cannula according to the invention is shown in Fig. 1 of the attached drawing. It comprises a curved outer tube 2 and an inner tube 3 that is slidably and removably fitted within the outer tube 2. The latter is to be inserted into the trachea of the patient through a neck hole that had been opened by laryngotracheotomy, a surgical operation known per se. To the open outer end of the inner tube 3 a box-like receptacle 1 made of plastics material is attached in order to prevent secretion discharge into the surroundings of the patient. As mentioned already, the box-like receptacle 1 is of the disposable kind, and has a closed front wall 11, a rear wall 12 facing the patient's chest, and bottom, top and side walls. Fig. 2 shows more clearly that the receptacle 1 further has two breathing holes 121 and a coupling hole 122 for the open outer end part of the inner tube 3. The coupling hole 122 is cut out from the rear wall 12 of the receptacle 1 so as to match both in shape and dimension with the open outer end part of the inner tube 3. More particularly, with the present embodiment as shown in Fig. 1 and 2, the two breathing holes 121 in the rear wall 12 of the receptacle 1 are arranged at both sides close to the coupling hole 122 in a manner that the breathing holes 121 and the coupling hole 122 form a single, suitably shaped opening within the rear wall 12. Thus, between the breathing holes 121 there is a wall portion 123, and a flange 31 of somewhat enlarged diameter of the inner tube 3 can be inserted into the receptacle 1 by slight temporary deformation of said wall portion 123 along an elongated area of the latter as indicated by dotted line in Fig. 2. Upon inserting the inner tube 3 into the outer tube 2, the upper and lower rear wall areas around the coupling hole 122 are clamped and held between flanges 31 and 21 of the inner tube 3 and the outer tube 2 respectively, as it may be clearly seen in Fig. 3. Exact positioning of the receptacle 1 with respect to the tubes 2 and 3 is effected by locating pins 44 that project from the flange 21 of the outer tube 2, and correspond with locating bores 124 in the wall portion 123 of the rear wall 12 shown in Fig. 2. The flange 31 of the inner tube 3 has projecting distance pins 33 embedded into its periphery. They extend into the interior of the receptacle 1 towards its front wall 11, and prevent possible excess deformation of the latter during breathing-in (as indicated in Fig. 3 by thin dotted line) that could cause a narrowing of the cross-sectional air flow area, and thus, could make breathing more difficult.

The trachea cannula according to the invention has also a fixing means 4 for securely but releasably attaching the receptacle 1 to the open end part of the inner tube 3. Said fixing means may be preferably though not exclusively a snap fastener in principle, for which a preferred embodiment is clearly shown in Fig. 3. As already mentioned above, both the outer tube 2 and the inner tube 3 have flanges 21, 31 at their open outer ends. The fixing means 4, designed and shaped as a snap fastener for example only, comprises an annular rim section 41 of the flange 31, which is insertable under a slight pressure (against tight fit and the action of a ring-shaped wire spring 43) into a matching bore section 42 within the flange 21.

As main component part of a further preferred embodiment of the trachea cannula according to the invention, Fig. 4 shows that the inner tube 3 and the receptacle 1 may also be designed and shaped as a single integral disposable component part made of plastics material. In addition to this, Fig. 4 indicates that with certain preferred embodiments, a pipe 34 of reduced diameter can be arranged within the inner tube 3. The pipe 34 has an inlet portion (not shown) that is in the proximity of the inner lower end of the inner pipe 3, and a pipe end 341 outside the receptacle 1. The pipe end 341 is suitable to be connected to a sucking device (not shown), preferably to a secretion pump, for positively removing excess secretion from the patient's trachea, especially during the initial period soon after operation. All embodiments of the device according to the invention may have another pipe 14 extending into the receptacle 1. The pipe 14 has an inlet portion 141 that is arranged in a suitable distance above the bottom wall of the receptacle. To a pipe end 142 outside of the receptacle 1 a sucking device (not shown), preferably a secretion pump, may be attached for temporary and positive discharge of the contents of the receptacle 1 without removing the latter from its working position.

**Claims**

1. A trachea cannula comprising a curved outer tube (2) insertable into the trachea of a patient through a neck hole opening surgically by laryngotracheotomy, an inner tube (3) slidably and removably fitted within the outer tube (2), and a box-like housing (1) provided with a rear wall (12) facing the patient's chest when the trachea cannular is in use, a front wall (11) and bottom, top and side walls, and formed with at least one breathing hole (121) and connected to the open outer end of the inner tube, characterised in that the box-like housing is constructed as a receptacle (1) adapted for collection of secretion and having a closed front wall (11), that the breathing hole (121) being formed as a free throughhole is located in the rear wall (12) or at least one side wall of the receptacle (1), and that the receptacle (1) either is of the disposable kind and simply removably attached to the inner tube (3) or is integrally formed with the inner tube (3) as a single disposable component part.

2. A trachea cannula according to claim 1, in the embodiment in which the receptacle (1) is simply removable attached to the inner tube (3), characterised in that a coupling hole (122) for the outer end part of the inner tube (3) is cut out in the rear wall (12) of the receptacle (1) in near proximity of the breaching hole (121).

3. A trachea cannula according to claim 2, characterised in that two breathing holes (121) are formed in the rear wall (12) of the receptacle (1), each at one side of the coupling hole (122) to form a single shaped opening with the coupling hole (122) in the rear wall (12) of the receptacle (1).

4. A trachea cannula according to claim 2 or 3, characterised by a fixing means (4) for secure but simply releasable attachment of the receptacle (1) to the open outer end part of the inner tube (3).

5. A trachea cannula according to claim 4, characterised in that each of the inner and outer tubes (2, 3) has a flange (21, 31) at its outer end, that the fixing means (4) is constructed as a snap fastener comprising an annular rim section (41) of the flange (31) of the inner tube (3), the rim section (41) being insertable under slight pressure against the action of a ring-shaped wire spring (43) into a matching bore section (42) in the flange (21) of the outer tube (2) to securely clamp portions of the rear wall (12) of the receptacle (1) between the flanges (21, 31) of the inner and outer tubes (2, 3).

6. A trachea cannula according to claim 5, characterised in that at least two distance pins (33) project from the flange (31) of the inner tube (3) towards the front wall (11) of the receptacle (1).

7. A trachea cannula according to one of claims 1 to 6, characterised in that a pipe (34) of reduced diameter is arranged within the inner tube (3) and has an inlet portion near the inner end of the inner tube (3) and a pipe end (341) extending out of the receptacle (1) and being adapted to be connected to a sucking device, preferably a secretion pump for positively removing excess secretion from the patient's trachea.

8. A trachea cannula according to one of claims 1 to 7, characaterised in that a pipe (14) extends into the receptacle (1) and has an inlet portion (141) in a distance above the bottom wall of the receptacle (1) and has a pipe end (142) outside the receptacle for connecting a sucking device, preferably a secretion pump for a temporary and positive discharge of the contents of the receptacle (1).

**Patentansprüche**

1. Tracheal-Kanüle mit einem gekrümmten Außenrohr (2), welches in die Luftröhre eines Patienten durch eine Halsöffnung hindurch einsetzbar ist, welche chirurgisch durch Laryngotracheotomie geöffnet wurde, einem Innenrohr (3), welches gleitend und lösbar in das Innenrohr (2) eingepaßt ist, und einem kastenartigem Gehäuse (1), welches mit einer Rückwand (12), die der Brust des Patienten zugewandt ist, wenn die Tracheal-Kanüle benutzt wird, einer Vorderwand (11) und Boden-, Deceken- und Seitenwände versehen ist und in welchem wenigstens eine Atmungsöffnung (121) angebildet ist und welches an dem offenen Außenende des Innenrohres angebracht ist, dadurch gekennzeichnet, daß das kastenartige Gehäuse als Behälter (1) ausgebildet ist, der zum Sammeln von Sekret geeignet ist und eine geschlossene Vorderwand (11) aufweist, daß die Atmungsöffnung (121), die als freie Durchgagnsöffnung ausgebildet ist, in der Rückwand (12) oder an wenigstens einer Seitenwand des Behälters (1) angeordnet ist und daß der Behälter (1) entweder vom Wegwerftyp und einfach lösbar an dem Innenrohr (3) befestigt ist oder mit dem Innenrohr (3) als einzige wegwerfbare Einheit einstückig ausgebildet ist.

2. Tracheal-Kanüle nach Anspruch 1 in der Aufführungsform, bei welcher der Behälter (1) an dem Innenrohr (3) einfach lösbar befestigt ist, dadurch gekennzeichnet, daß eine Kupplungsöffnung (122) für den äußeren Endteil des Innenrohres (3) in der Rückwand (12) des Behälter (1) nahe an der Atmungsöffnung (121) ausgeschnitten ist.

3. Tracheal-Kanüle nach Anspruch 2, dadurch gekennzeichnet, daß zwei Atmungsöffnungen (121) in der Rückwand (12) des Behälters (1) jeweils zu einer Seite der Kupplungsöffnung (122) ausgebildet sind und mit der Kupplungsöffnung (122) in der Rückwand (12) des Behälters (1) eine einzige Öffnung bilden.

4. Tracheal-Kanüle nach Anspruch 2 oder 3, gekennzeichnet durch eine Befestigungseinrichtung (4) zum sicheren, jedoch einfach lösbaren Befestigen des Behälters (1) an dem offenen Außenendteil des Innenrohres (3).

5. Tracheal-Kanüle nach Anspruch 4, dadurch gekennzeichnet, daß das Innenrohr (2) und das Außenrohr (3) jeweils einen Flansch (21, 31) an seinem Außenende aufweist, daß die Befestigungseinrichtung (4) als Schnappverschluß mit einem Ringbundabschnitt (41) dês Flansches (31) des Innenrohres (3) auasgebildet ist, wobei der Ringbundabschnitt (41) unter leichtem Druck gegen die Wirkung einer ringförmigen Drahtfeder (43) in einen angepaßten Bohrungsabschnitt (42) in dem Flansch (21) des Außenrohres (2) einführbar ist, um Teil der Rückwand (12) des Behälters (1) zwischen den Flanschen (21, 31) des Innenrohres (3) und des Außenrohres (2) sicher einzuklemmen.

6. Tracheal-Kanüle nach Anspruch 5, dadurch gekennzeichnet, daß wenigstens zwei Distanzstifte (33) von dem Flansch (31) des Innenrohres (3) zu der Vorderwand (11) des Behälters (1) hin vorstehen.

7. Tracheal-Kanüle nach einem der Ansprüche 1 bis 6, dadurch gekennzeichnet, daß ein Rohr (34) reduzierten Durchmessers in dem Innenrohr (3) angeordnet ist und einen Einlaßteil nahe des Innenendes des Innenrohres (3) und ein Rohrende (341) aufweist, welches sich aus dem Behälter (1) heraus erstreckt und an eine Saugvorrichtung, vorzugsweise eine Sekretpumpe, zum positiven Abführen von übermäßigen Sekret aus der Luftröhre des Patienten anschließbar ist.

8. Tracheal-Kanüle nach einem der Ansprüche 1 bis 7, dadurch gekennzeichnet, daß ein Rohr (14) sich in den Behälter (1) ertreckt und einen Einlaßteil (141) in einem Abstand über der Bodenwand des Behälters (1) sowie ein Rohrende (142) außerhalb des Behälters zum Anschließen einer Saugvorrichtung, vorzungsweise einer Sekretpumpe, zum zeitweisen und positiven Abführen des Inhalts des Behälters (1) aufweist.

**Revendications**

1. Une canule trachéale comprenant un tube extérieur incurvé (2) pouvant être inséré dans la trachée d'un patient par l'intermédiaire d'un trou ouvert chirurgicalement dans le cou par laryngotrachéotomie, in tube intérieur (3) monté de façon coulissante et amovible à l'intérieur du tube extérieur (2) et un carter en forme de boîte (1) pourvu d'une paroi arrière (12) dirigée vers la poitrine du patient lorsque la canule trachéale est en service d'une paroi avant (11) et de parois de fond, supérieure et latérales, et comportant au moins un trou de respiration (121) et relié à l'extrémité extérieure ouverte du tube intérieur, caractérisée en ce que le carter en forme de boîte est réalisé sous forme d'un réceptacle (1) adapté pour collecter des sécrétions et comportant une paroi avant fermée (11), en ce que le trou de respiration (121) est réalisé sous la forme d'un trou traversant libre que est ménagé dans la paroi arrière (12) ou au moins dans une paroi latérale du réceptacle (1), et en ce que le réceptacle (1) est soit du type jetable et est fixé simplement de façon amovible sur le tube intérieur (3) ou bien est intégré au tube intérieur (3) sous forme d'un seul composant jetable.

2. Une canule trachéale selon la revendication 1, dans la réalisation dans laquelle le réceptacle (1) est simplement fixé de façon amovible sur le tube intérieur (3), caractérisée en ce qu'un trou d'accouplement (122) pour la partie extrême extérieure du tube intérieur (3) est ménagée dans la paroi arrière (12) du réceptacle (1) à proximité du trou de respiration (121).

3. Une canale trachéale selon la revendication 2, caractérisée en ce que deux trous de respiration (121) sont formés dans le paroi arrière (12) du réceptacle (1), chacun situé d'un côté du trou d'accouplement (122) pour former une seule ouverture profilée avec le trou d'accouplement (122) ménagé dans le paroi arrière (12) du réceptacle (1).

4. Une canule trachéale selon la revendication 2 ou 3, caractérisé par un moyen de fixation (4) pour relier, de façon sûre mais séparable d'une manière simple, le réceptacle (1) à la partie extrême extérieure ouverte du tube intérieur (3).

5. Une canule trachéale selon la revendication 4, caractérisée en ce que chacun des tubes interieur et extérieur (2, 3) comporte, à son extrémité extérieure, une collerette (21, 31), en ce que le moyen de fixation (4) est réalisé sous forme d'une attache emboîtable comportant une bordure annulaire (41) de la collerette (31) du tube intérieur (3), la bordure (41) pouvant être insérée sous une légère pression en opposition à l'action d'une ressort annulaire en fil (43) dans une partie correspondante d'alésage (42) ménagée dans la collerette (21) du tube extérieur (2) afin de bloquer solidement des parties de la paroi arrière (12) du réceptacle (1) entre les collerettes (21, 31) des tubes intérieur et extérieur (2, 3).

6. Une canule trachéale selon la revendication 5, caractérisée en ce qu'au moins deux broches d'espacement (33) font saillie de la collerette (31) du tube intérieur (3) en direction de la paroi avant (11) du réceptacle (1).

7. Une canale trachéale selon une des revendications 1 à 6, caractérisée en ce qu'un tube (34) de diamètre réduit est disposé à l'intérieur du tube intérieur (3) et comporte une partie d'entrée proche de l'extrémité intérieure du tube intérieur (3) et une extrémité du tube (341) sortant du réceptacle (1) et apte à être reliée à un dispositif d'aspiration, de préférence une pompe à sécrétion permettant d'enlever efficacement l'excès de sécrétion de la trachée du patient.

8. Une canule trachéale selon une des revendicatons 1 à 7, caractérisée en ce qu'un tube (14) pénètre dans le réceptacle (1) et comporte une partie d'entrée (141) placée à distance et audessus de la paroi de fond due réceptacle (1) et comporte une extrémité de tube (142) placée à l'extérieur du réceptacle pour la liaison avec un dispositif d'aspiration, de préference une pompe à sécretion en vue d'une décharge temporaire et effice du contenu de réceptacle (1).

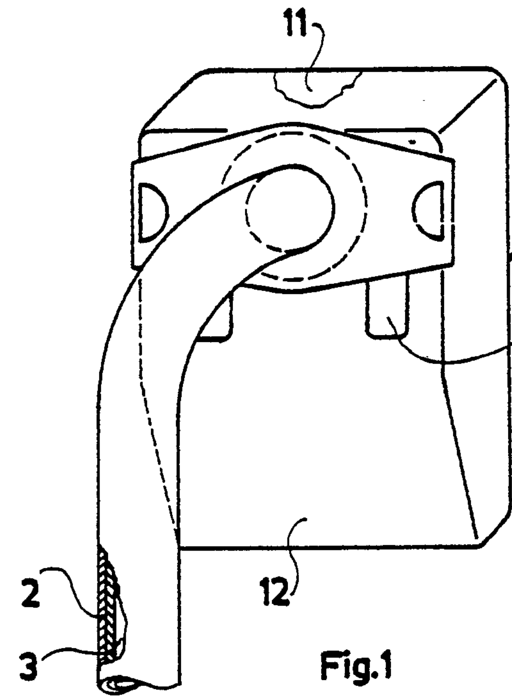


Fig.1

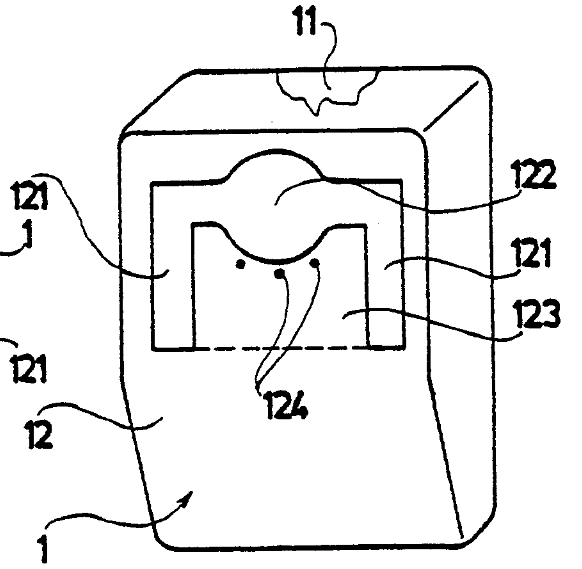


Fig.2

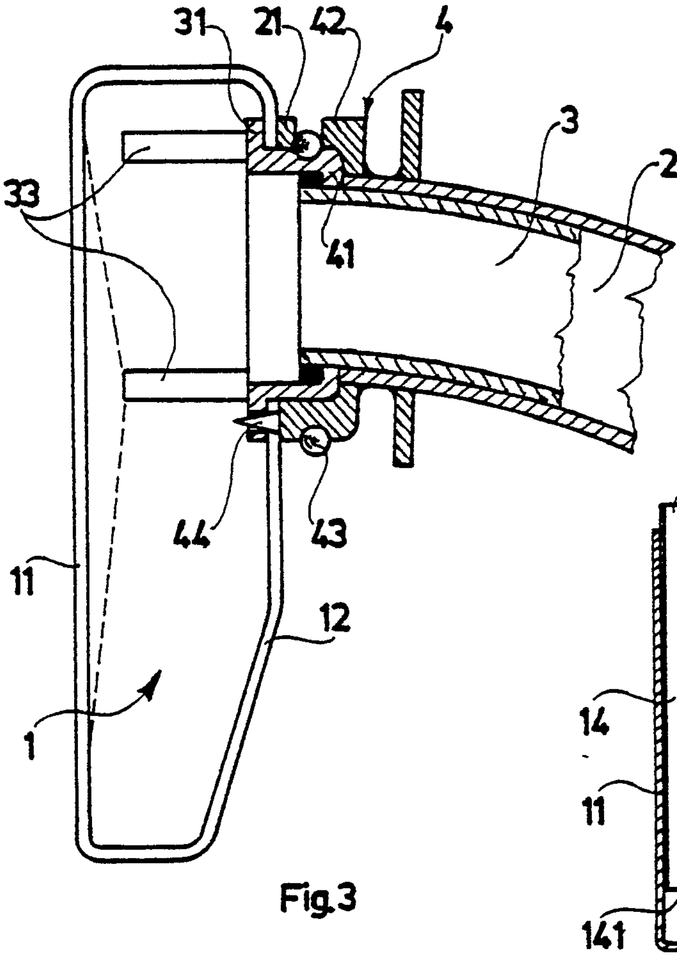


Fig.3

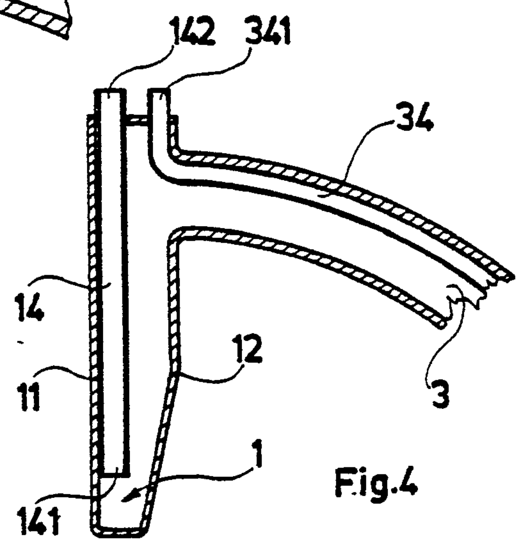


Fig.4